# EUROPÄISCHE PATENTANMELDUNG

(11) **EP 0 527 431 A1**
(43) Veröffentlichungstag der Anmeldung: **17.02.1993**
(21) Anmeldenummer: 92113354.2
(22) Anmeldetag: 05.08.1992
(51) Int. Cl.: A61B 17/44

(54) **Griffteil für eine Saugglocke zur Geburtshilfe**

(30) Priorität: 08.08.1991 DE 4126331
(71) Anmelder: King, Siegfried, Dr. med. Ing. grad.,, D-82061 Neuried (DE)
(72) Erfinder: King, Siegfried, Dr. med. Ing. grad.,, D-82061 Neuried (DE)
(74) Vertreter: von Puttkamer, Nikolaus, Dipl.-Ing. Patentanwälte Haft, von Puttkamer Berngruber, Czybulka

(57) **Zusammenfassung**

Die Erfindung betrifft ein Griffteil für eine Saugglocke zur Geburtshilfe mit einem in der Längsrichtung des Griffteiles verlaufenden Innendurchgang (11), wobei ein Endbereich (4) des Griffteiles (2) an einem Rohrstutzen (8) der Saugglocke (1) ansetzbar und der andere Endbereich (16) des Griffteiles (2) mit einer zu einer Vakuumpumpe führenden Verbindungsleitung verbindbar ist. Der eine Endbereich (4) ist derart mit dem Rohrstutzen (8) der Saugglocke (1) verbunden, daß das Griffteil (2) in Bezug auf den Rohrstutzen (8) der Saugglocke (1) verdrehbar ist.

## Beschreibung

Die Erfindung betrifft ein Griffteil für eine Saugglocke zur Geburtshsilfe nach dem Oberbegriff des Patentanspruches 1.

Aus dem europäischen Patent 212 475 geht ein Vakuum-Extraktor zur Geburtshilfe hervor, der ein Griffteil aufweist, dessen der Saugglocke zugewandter Endbereich über eine Kugellageranordnung mit der Saugglocke derart verbunden ist, daß er von der Saugglocke entkoppelt ist, so daß er eine Kreiselbewegung ausführen kann. Genauer gesagt wird durch das Griffteil und die Kugellageranordnung erreicht, daß das Griffteil in Bezug auf die Saugglocke in einem vorgegebenen Winkelbereich nach allen Richtungen in Bezug auf die Längsachse der Saugglocke weitgehend reibungsfrei verschwenkbar ist. Ferner wird durch die Kugellageranordnung erreicht, daß das Griffteil in Bezug auf die Saugglocke um die Längsachse derselben verdreht werden kann. Ein Problem des bekannten Griffteiles mit der Kugellageranordnung besteht darin, daß es relativ kompliziert aufgebaut und daher nur schwer und mit großen Kosten realisierbar ist.

Die Aufgabe der vorliegenden Erfindung besteht daher darin, ein Griffteil der eingangs genannten Art dahingehend zu verbessern, daß es vergleichsweise einfach aufgebaut und daher kostengünstig realisierbar ist, wobei es eine Verschwenkung nach allen Richtungen in Bezug auf die Längsachse der Saugglocke und eine Verdrehung um die Längsachse in Bezug auf die Saugglocke zuläßt.

Diese Aufgabe wird durch ein Griffteil für eine Saugglocke der eingangs genannten Art gelöst, das durch die in dem kennzeichnenden Teil des Patentanspruches 1 angegebenen Merkmale gekennzeichnet ist.

Der wesentliche Vorteil des erfindungsgemäßen Griffteiles besteht darin, daß es bei einem äußerst einfachen Aufbau eine Drehbewegung des Griffteiles um die Längsachse in Bezug auf die Saugglocke und eine Verschwenkung des Griffteiles in Bezug auf die Längsachse der Saugglocke zuläßt. Wegen des besonders einfachen Aufbaues kann das erfindungsgemäße Griffteil äußerst kostengünstig realisiert werden. Vorteilhafterweise ist das erfindungsgemäße Griffteil gegenüber der Längsachse der Saugglocke nach allen Richtungen um einen Winkel von bis zu maximal 90° verschwenkbar. Die Drehung des Griffteiles um die Längsachse der Saugglocke kann beliebig in einem Winkelbereich von 360° erfolgen.

Ein wesentlicher Vorteil besteht darin, daß das erfindungsgemäße Griffteil besonders einfach von der Saugglocke abtrennbar ist, so daß die Saugglocke vom Griffteil getrennt gereinigt und sterilisiert werden kann.

Vorteilhafterweise weist das erfindungsgemäße Griffteil einen Innendurchgang auf, über den die Evakuierung der Saugglocke erfolgt und durch den beliebige Meßdaten zur Geburtsüberwachung vom Kopf eines Kindes zu Meßgeräten übertragen werden können.

Besonders vorteilhaft und preisgünstig wird das vorliegende Griffteil in einer Form aus Silikon einstückig gegossen.

Weitere vorteilhafte Ausgestaltungen der Erfindung gehen aus den Unteransprüchen hervor.

Im folgenden werden die Erfindung und deren Ausgestaltungen im Zusammenhang mit der Fig. näher erläutert.

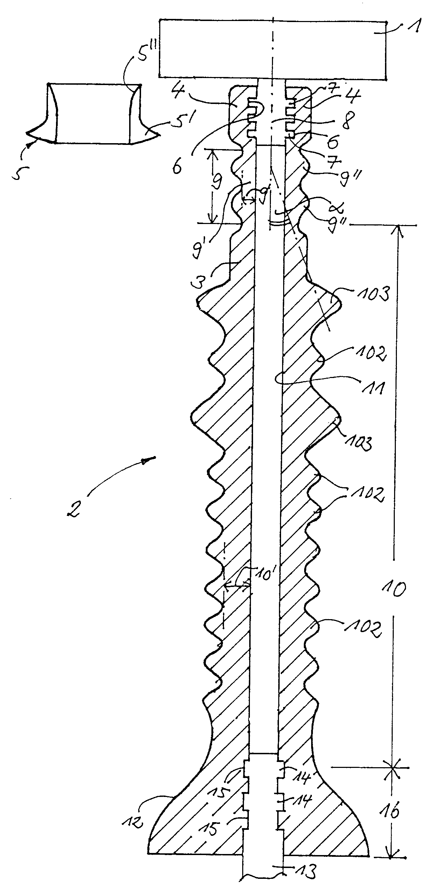

In der Figur bezeichnet das Bezugszeichen 1 eine bekannte Saugglocke, die eine übliche Saugglocke mit einer Vakuumkammer oder eine Saugglocke mit mehreren, getrennt voneinander evakuierbaren Vakuumkammern sein kann. Die Saugglocke 1 weist einen Rohrstutzen 8 auf, über den die Evakuierung der Saugglocke 1 erfolgt. Auf den Rohrstutzen 8 ist der der Saugglocke 1 zugewandte Endbereich des vorliegenden Griffteiles 2 aufsetzbar. Das Griffteil 2 besteht zumindest in dem an die Saugglocke 1 anschließenden Bereich aus einem flexiblen Material, das seine Auslenkung in Bezug auf die Längsachse der Saugglocke 1 nach allen Richtungen um einen Winkelα zuläßt, der maximal 90° beträgt. Vorzugsweise handelt es sich bei dem flexiblen Material um Silikon.

Das Griffteil 2 besteht vorzugsweise insgesamt aus einem länglichen, rohrartigen Silikonteil, das einen Innendurchgang 11 umschließt. In diesen Innendurchgang 11 ist an der der Saugglocke 1 zugewandten Seite des Griffteiles 2 der Rohrstutzen 8 der Saugglocke 1 einschiebbar bzw. einsetzbar. Um die Verbindung zwischen der Saugglocke 1 und dem Griffteil 2 auch dann zu gewährleisten, wenn bei der Anwendung der Saugglocke 1 über das Griffteil 2 auf die Saugglocke 1 ein Zug ausgeübt wird, weist die Innenwandung des der Saugglocke 1 zugewandten Endbereiches 4 des Griffteiles 2 vorzugsweise wenigstens eine Haltenut 6 auf, in die ein zweckmäßigerweise ringförmiger Haltevorsprung 7 des Rohrstutzens 8 der Saugglocke 1 eingreift, wenn das Griffteil 2 an der Saugglocke 1 befestigt ist. Um eine besondes gute Verbindung zwichen der Saugglocke 1 und dem Griffteil 2 sicherzustellen, sind an der Innenwandung des Innendurchganges 11 des Endbereiches 4 vorzugsweise mehrere, insbesondere drei voneinander beabstandete Haltenuten 6 vorgesehen, in die drei voneinander entsprechend beabstandete Haltevorsprünge 7 des Rohrstutzens 8 eingreifen. Besonders vorteilhaft ist es, daß die Haltevorsprünge 7 an den Rohrstutzen 8 von auf dem Markt befindlichen Saugglocken 1 bereits vorhanden sind.

Es wird darauf hingewiesen, daß es auch denkbar wäre, die Haltenuten 6 in der Oberfläche des Rohrstutzens 8 und die Haltevorsprünge 7 an der Innenwandung des Innendurchganges 11 des Endbereiches 4 vorzusehen.

Durch die beschriebene Verbindung zwischen dem Endbereich 4 und dem Rohrstutzen 8 wird sichergestellt, daß der Endbereich 4 in Bezug auf den Rohrstutzen 8 um die Längsachse des Rohrstutzens 8 frei drehbar ist.

Um zu verhindern, daß der Endbereich 4 bei der Ausübung eines besonders großen Zuges über das Griffteil 2 auf die Saugglocke 1 vom Rohrstutzen 8 durch Aufweiten der in dem flexiblen Material des Endbereiches 4 vorgesehenen Haltenuten 6 losgelöst wird, ist vorzugsweise ein Konterring 5 vorgesehen, der nach der Montage des Endbereiches 6 am Rohrstutzen 8 über den Endbereich 4 geschoben wird. Zu diesem Zweck ist der Innendurchmesser des Konterringes 5 so beschaffen, daß er im montierten Zustand die Außenfläche des Endbereiches 4 fest umschließt, so daß auch bei Ausübung einer großen Zugkraft das Material des Endbereiches 6 fest an den Haltevorsprüngen 7 des Rohrstutzens 8 anliegt. Der Konterring 5 weist vorzugsweise die Form eines Rohrteiles auf, an dessen der Saugglocke 1 abgewandten Ende ein nach außen ragender Flansch 5' angeordnet ist, der ein Abziehen des Konterringes 5 vom Endbereich 4 erleichtert. Um ein Aufschieben des Konterringes 5 auf den Endbereich 4 zu erleichtern, weist dieser einen vorderen Bereich 5'' auf, in dem sich sein Innendurchmesser von einem Wert der größer ist als der Außendurchmesser des Endbereiches 4 auf den genannten Innendurchmesser verjüngt.

An den Endbereich 4 schließt sich ein Schwenkbereich 9 mit einer Wandstärke 9' an, die eine Verbiegung gegenüber der Längsachse der Saugglocke 1 zuläßt. Dieser Schwenkbereich 9 ermöglicht, daß die Ebene des Saugrandes der Saugglocke 1 etwa parallel zur Längsachse des Griffteiles 2 elastisch verschwenkt werden kann, so daß die Saugglocke 1 besonders leicht in den Geburtskanal eingeführt werden kann. Nach dem Anlegen der Saugglocke 1 ermöglicht der Schwenkbereich 9 eine automatische Anpassung an den Geburtskanal in Abhängigkeit von den über das Griffteil 2 ausgeübten Zugkräften. Vorzugsweise beträgt die Länge dieses Schwenkbereiches 9 etwa 3,5 cm bei einer Wandstärke 9' von etwa 3 mm bei einer Ausführung des Griffteiles 2 aus Silikon. Um zu verhindern, daß bei einer Abbiegung des Schwenkbereiches um große Winkel der Innendurchgang 11 im Schwenkbereich 9 abgeknickt wird, so daß die Verbindung der Saugglocke 1 zu einer externen Vakuumpumpe unterbrochen werden könnte und daß durch den Innendurchgang 11 verlaufende Meßleitungen beschädigt werden könnten, weist der Schwenkbereich 9 an seinem Außenumfang vorzugsweise voneinander beabstandete, ringförmige Stütznoppen 9'' auf, die bei großen Auslenkwinkeln, z.B. ab etwa 60° aneinander zur Anlage kommen und sicherstellen, daß der Innendurchgang 11 nicht in einer unerwünschten Weise verschlossen bzw. abgeknickt wird. An den Schwenkbereich 9 schließt sich an der der Saugglocke 1 abgewandten Seite ein Bereich 10 des Griffteiles 2 an, dessen Wandstärke 10' größer als die Wandstärke 9' des Schwenkbereiches 9 ist, so daß in diesem Bereich im wesentlichen keine Verbiegung gegenüber der Längsachse des Griffteiles 2 erfolgt. An der Außenseite des Bereiches 10 können voneinander beabstandet ringförmige Verstärkungsnoppen 102 vorgesehen sein, die die Steifigkeit des Bereiches 10 erhöhen. An der der Saugglocke 1 zugewandten Seite des Bereiches 10 können voneinander beabstandete ringförmige nach außen ragende Ziehnoppen 103 angeordnet sein, wobei bei der Handhabung des Griffteiles 2 der Zeigefinger und der Daumen des Arztes in den Bereich zwischen diesen beiden Ziehnoppen 103 eingreifen.

Vorzugsweise erweitert sich der der Saugglocke 1 abgewandte Endbereich 16 des Griffteiles 2 wulstförmig nach außen, so daß bei der Handhabung des Griffteiles 2 der Handballen an der Außenfläche 12 dieses wulstförmigen Bereiches 16 abstützend anliegt. Zur Verbindung des Griffteiles 2 mit einer externen Vakuumpumpe wird in den Innendurchgang 11 des Bereiches 12 ein mit einer Vakuumleitung (nicht dargestellt) verbindbarer Rohrstutzen 13 eingeführt, der zur Herstellung einer festen Verbindung mit dem Griffteil 2 Haltevorsprünge 14 aufweisen kann, die in entsprechende Haltenuten 15 eingreift, die sich in der Innenwandung des Bereiches 12 befinden. Vorzugsweise sind mehrere, beispielsweise drei voneinander beabstandete Haltevorsprünge 14 und Haltenuten 15 vorgesehen.

Das beschriebene Griffteil 2 ist besonders einfach einstückig in einer Form aus vorzugsweise einem Silikonmaterial herstellbar. Der Konterring 5 besteht beispielsweise aus einem Metallmaterial, vorzugsweise aus Aluminium oder Messing.

Es wird darauf hingewiesen, daß die axiale Länge des Schwenkbereiches 9 so bemessen ist, daß der Konterring 5 lose in dem Schwenkbereich 9 gehalten wird, wenn er vom Endbereich 4 abgezogen ist. Zu diesem Zweck ist die Wandstärke im Schwenkbereich 9 kleiner als die Wandstärke im Endbereich 4 und im sich an der anderen Seite anschließenden Bereich des Griffteiles 2. Der Konterring 5 wird daher im Schwenkbereich 9 unverlierbar gehalten und ist, wenn er sich in dieser Lage befindet leicht reinigbar.

Der der Saugglocke 1 zugewandte Ziehnoppen 103 kann bei am kindlichen Kopf angelegter Saugglocke 1 ein Orientierungsmaß für den Arzt im Hinblick auf die Höhe der Lage des Kopfes im mütterlichen Becken sein, weil sein Abstand zum Saugrand der Saugglocke 1 so bemessen ist, daß er bei der richtigen Höhe des Kopfes gerade vor der Vulver der Entbindenden zu liegen kommt.

Es wird darauf hingewiesen, daß der dem Endbereich 16 zugewandte Ziehnoppen 103 entfallen kann. In diesem Fall liegt der Zeigefinger des Arztes an dem verbleibenden Ziehnoppen 103 an, der vorzugsweise die zuvor genannte Anzeigefunktion erfüllt.

Als das Abknicken bei großen Abwinkelungen im Schwenkbereich 9 verhindernde Einrichtung können an der Stelle der genannten beiden Stütznoppen 9'' auch andere Mittel vorhanden sein. Beispielsweise kann nur ein Stütznoppen 9'' vorgesehen sein, der beim Auslenken um große Winkel an der einen oder anderen Schulter zur Anlage gelangt, die am Übergang zwischen der kleinen Wandstärke des Schwenkbereiches 9 und den größeren Wandstärken des Endbereiches 4 sowie des Hauptbereiches 10 gebildet sind. Von Bedeutung ist es lediglich, daß durch die genannte Einrichtung ein Abknicken des Innendurchganges 11 im Schwenkbereich 9 mit den weiter oben beschriebenen Nachteilen verhindert wird.

## Patentansprüche

1. Griffteil für eine Saugglocke zur Geburtshilfe mit einem in der Längsrichtung des Griffteiles verlaufenden Innendurchgang (11), wobei ein Endbereich (4) des Griffteiles (2) an einem Rohrstutzen (8) der Saugglocke (1) ansetzbar ist und der andere Endbereich (16) des Griffteiles (2) mit einer zu einer Vakuumpumpe führenden Verbindungsleitung verbindbar ist, dadurch gekennzeichnet, daß der eine Endbereich (4) derart mit dem Rohrstutzen (8) der Saugglocke (1) verbunden ist, daß das Griffteil (2) in Bezug auf den Rohrstutzen (8) der Saugglocke (1) verdrehbar ist.

2. Griffteil nach Anspruch 1, dadurch gekennzeichnet, daß der eine Endbereich (4) in seiner Innenwandung des Innendurchganges (11) wenigstens eine Haltenut (6) aufweist, in die ein Haltevorsprung (7) des Rohrstutzens (8) eingreift.

3. Griffteil nach Anspruch 2, dadurch gekennzeichnet, daß die Haltnut (6) und der Haltevorsprung (7) ringförmig ausgebildet sind.

4. Griffteil nach Anspruch 2 oder 3, dadurch gekennzeichnet, daß entlang der Längsachse des einen Endbereiches (4) voneinander beabstandet mehrere Haltenuten (6) angeordnet sind, in die entsprechend voneinander beabstandete Haltesprünge (7) des Rohrstutzens (8) eingreifen.

5. Griffteil nach einem der Ansprüche 1 bis 4, dadurch gekennzeichnet, daß an den einen Endbereich (4) ein Schwenkbereich (9) anschließt, in dem das Griffteil (2) aus der Längsachse des Griffteiles (2) verschwenkbar ist.

6. Griffteil nach Anspruch 5, dadurch gekennzeichnet, daß wenigstens der eine Endbereich (4) und der Schwenkbereich (9) des Griffteiles (2) aus einem flexiblen Material bestehen.

7. Griffteil nach Anspruch 6, dadurch gekennzeichnet, daß das flexible Material Silikon ist.

8. Griffteil nach einem der Ansprüche 5 bis 7, dadurch gekennzeichnet, daß die Wandstärke (9') des Schwenkbereiches (9) kleiner ist als die Wandstärke des einen Endbereiches (4) und als die Wandstärke (10') des an den Schwenkbereich (9) an der dem einen Endbereich (4) abgewandten Seite angrenzenden Hauptbereiches (10) des Griffteiles (2).

9. Griffteil nach einem der Ansprüche 5 bis 8, dadurch gekennzeichnet, daß im Schwenkbereich (9) wenigstens zwei in der Längsrichtung voneinander beabstandete Stütznoppen (9'') vorgesehen sind, die beim Abbiegen im Schwenkbereich (9) um große Winkel stützend aneinander zur Anlage gelangen, um ein Abknicken des Griffteiles (2) im Schwenkbereich (9) zu verhindern.

10. Griffteil nach einem der Ansprüche 1 bis 9, dadurch gekennzeichnet, daß der Hauptbereich (10) in der Längsrichtung voneinander beabstandete ringförmige Verstärkungsnoppen (102) aufweist.

11. Griffteil nach einem der Ansprüche 1 bis 10, dadurch gekennzeichnet, daß der Hauptbereich (10) an seiner dem einen Endbereich (4) zugewandten Seite wenigstens einen ringförmigen Ziehnoppen (103) aufweist, an dem beim Gebrauch des Griffteiles (2) der Zeigefinger des Arztes angreifen kann.

12. Griffteil nach Anspruch 11, dadurch gekennzeichnet, daß in der Längsrichtung voneinander beabstandet zwei Ziehnoppen (103) vorgesehen sind, die derart voneinander beabstandet sind, daß beim Gebrauch des Griffteiles (2) der Daumen und der Zeigefinger des Arztes in den Zwischenraum zwischen den beiden Ziehnoppen (103) eingreifen können.

13. Griffteil nach Anspruch 11 oder 12, dadurch gekennzeichnet, daß die Ziehnoppen (103) weiter nach außen ragen als die Verstärkungsnoppen (102).

14. Griffteil nach einem der Ansprüche 1 bis 13, dadurch gekennzeichnet, daß der andere Endbereich (16) eine in Richtung auf die dem einen Endbereich (4) abgewandte Seite wulstförmig nach außen verlaufende Auflagefläche (12) für den Handballen des Arztes beim Gebrauch des Griffteiles (2) aufweist.

15. Griffteil nach einem der Ansprüche 1 bis 14, dadurch gekennzeichnet, daß im anderen Endbereich (16) in der Innenfläche des Innendurchganges (11) wenigstens eine Nut (15) vorgesehen ist, in die ein mit einer Verbindungsleitung zu einer externen Vakuumpumpe führendes Rohrteil (13) mit einem Vorsprung (14) eingreift.

16. Griffteil nach Anspruch 15, dadurch gekennzeichnet, daß die Nut (15) und der Vorsprung (14) ringförmig ausgebildet sind.

17. Griffteil nach Anspruch 15 oder 16, dadurch gekennzeichnet, daß in der Längsrichtung des anderen Endbereiches (16) voneinander beabstandet mehrere Nuten (15) vorgesehen sind, die in die entsprechend voneinander beabstandete Vorsprünge (14) des Rohrteiles (13) eingreifen.

18. Griffteil nach einem der Ansprüche 1 bis 17, dadurch gekennzeichnet, daß es einen Konterring (5) aufweist, der auf den einen Endbereich (4) aufschiebbar ist und dessen Innendurchmesser so bemessen ist, daß der Endbereich (4) gegen den Rohrstutzen (8) derart gedrückt wird, daß die Haltevorsprünge (7) in den Haltenuten (6) festgehalten werden.

19. Griffteil nach Anspruch 18, dadurch gekennzeichnet, daß der Konterring an seiner der Saugglocke (1) abgewandten Seite einen nach außen ragenden Ziehflansch (5') aufweist.

20. Griffteil nach Anspruch 18 oder 19, dadurch gekennzeichnet, daß der Innendurchgang des Konterringes (5) an seiner Saugglocke (1) zugewandten Seite sich von einem Durchmesser, der größer ist als der Innendurchmesser des Konterringes (5) zu dem Innendurchmesser des Konterringes (5) verjüngt.

21. Griffteil nach einem der Ansprüche 18 bis 20, dadurch gekennzeichnet, daß die axiale Länge des Konterringes (5) kleiner ist als diejenige des Schwenkbereiches (9), so daß der Konterring (5) im Schwenkbereich (9) unverlierbar gehalten wird.

22. Griffteil nach einem der Ansprüche 1 bis 21, dadurch gekennzeichnet, daß es ingesamt aus einem flexiblen Material besteht.

23. Griffteil nach Anspruch 20, dadurch gekennzeichnet, daß das Material Silikon ist.

24. Griffteil nach einem der Ansprüche 11 bis 23, dadurch gekennzeichnet, daß der dem einen Endbereich (4) zugewandte Ziehnoppen (103) vom Saugrand der Saugglocke (1) so beabstandet ist, daß er bei der richtigen Höhe des Kopfes im mütterlichen Becken etwa gerade vor der Vulver der Entbindenden zu liegen kommt.
